Europäisches Patentamt

European Patent Office

Office européen des brevets

(19)

(11) Publication number: **0 371 723**
**A2**

(12) **EUROPEAN PATENT APPLICATION**

(21) Application number: **89312282.0**

(22) Date of filing: **27.11.89**

(51) Int. Cl.⁵: **G03F 7/031, C07C 225/22, C07C 49/784**

(30) Priority: **01.12.88 US 278554**

(43) Date of publication of application:
**06.06.90 Bulletin 90/23**

(84) Designated Contracting States:
**AT BE CH DE ES FR GB GR IT LI LU NL SE**

(71) Applicant: **Polychrome Corporation**
**137 Alexander Street**
**Yonkers New York 10702(US)**

(72) Inventor: **Rowe, William**
**RR1 Box 103**
**Califon New Jersey(US)**
Inventor: **Shah, Ajay**
**241 W. Grand St.-C5**
**Elizabeth New Jersey(US)**

(74) Representative: **Lawrence, Peter Robin**
**Broughton et al**
**GILL JENNINGS & EVERY 53-64 Chancery**
**Lane**
**London WC2A 1HN(GB)**

(54) **Photocurable compositions and methods.**

(57) Photocurable compositions which comprise a light sensitive composition in combination with at least one initiator therefor are improved by the use of dibenzoyl benzene as the photoinitiator. In a preferred combination, para-dibenzoyl benzene is employed in combination with para-di(4-dimethylaminobenzoyl) benzene. The photocurable compositions can be used for various purposes including preparation of lithographic printing plates.

EP 0 371 723 A2

## PHOTOCURABLE COMPOSITIONS AND METHODS

Light sensitive resinous compositions are well known and are used for a wide variety of purposes including, for example, making lithographic printing plates, multicolor proofs, visual aids and printed circuits, and the like. These compositions generally comprise a photocurable component which may be a monomer, polymer or various combinations thereof together with an initiator. When irradiated with a suitable light source, free radicals are generated to cause polymerization, cross-linking or some combination thereof.

For example, lithographic printing plates have a coating of the light sensitive composition adhering to a suitable base sheet material such as an aluminum sheet. When the light sensitive coating is applied to the base sheet by the manufacturer, the plate is referred to as a "presensitized plate." When the coating is applied by the lithographic or trade platemaker, the plate is referred to as a "wipe-on" plate. As desired, the coated plate can be utilized to reproduce directly the image to which it is exposed, in which the case the plate is termed "positive-acting" or to reproduce a complimentary image, in which case the plate is designated "negative-acting". In either event, the image areas of the developed plate are relatively oleophilic and the non-image areas are relatively hydrophilic.

A popular photoinitiator in wide use today is benzophenone. Since it is particularly adapted for curing the surface of the photosensitive composition, the benzophenone is frequently employed in combination with a through-curing photoinitiator such as Michler's ketone. Unfortunately, benzophenone is known to sublime out of thin films with the passage of time which materially affects the speed at which the photocuring reaction occurs and therefore requires adjustments in the curing conditions. The loss of the photoinitiator occurs upon storage of presensitized plates. Accordingly, there is a continuing need for a photoinitiator which does not result in degradation of the photocuring properties of the composition in which it is incorporated with the passage of time.

It is accordingly the object of this invention to provide new light sensitive compositions containing photoinitiators which provide increased storage stability. This and other objects of the invention will become apparent to those skilled in this art from the following detailed description.

This invention relates to photocurable compositions and their use and more particularly to photocurable compositions comprising a light sensitive composition and a photoinitiator in which the photoinitiator comprises dibenzoyl benzene, which may be substituted or unsubstituted.

The present invention is applicable to all photocurable compositions which comprise a light sensitive composition in combination with a photoinitiator. Such compositions are used, for example, to form lithographic printing plates, proofs for multi-colored printing, visual aids, printed circuits, UV curable 100% non-volatile coatings and adhesives, UV curable printing inks, and the like. For convenience only, the following description will focus on lithographic printing plates although it will be recognized that the invention is not limited thereto.

A lithographic printing plate generally has a coating of a light curable composition adhered to a suitable base sheet material. Any material known and commonly used as a lithographic base surface for printing plates can be used in the present invention and include those made from paper, fabric, synthetic resins, and especially in the case of long running dimensionally stable plates, from metals. Commonly used metals include steel, stainless steel, zinc, aluminum, copper, chromium, and for a variety of reasons well known in the art, aluminum in the form of a sheet. The base may have been treated in a number of ways known in the art to enhance its character as a lithographic surface such as, for instance, graining a metal plate either mechanically or chemically as described in U. S. Patents 2,882,153 and 2,882,154, to enable better bonding of the coating subsequently applied. An aluminum sheet can be electrolytically anodized to form a harder, more resistant and abrasion resistant surface as described in U.S. Patent 3,440,050 and British patent 1,235,863. If desired, an intermediate coating can be placed on the base coating to provide a firmer bond between the surface and the resulting photocurable composition as disclosed, for example, in U.S. Patents 2,946,683 and 3,160,506,

In general, the light curable composition can be any material or combination of materials which is polymerizable and/or cross-linkable through a free radical mechanism, hydrogen abstraction or complex mechanism. Photocurable materials include acrylic or methacrylic acids esters of alcohols, polyhydric alcohols exemplified by trimethylolpropane triacrylate, pentaerythritol triacrylate and pentaerythritol pentacetate, dipentaerythritol hexamethacetate, propylene glycol dimethacrylate, and bisacrylates of oxyethylated bisphenol A derivatives. Also suitable are the low molecular weight, urethane group containing acrylates and methacrylates obtained by reaction with bivalent or polyvalent isocyanates.

For lithographic and printing plate purposes, the light sensitive composition described in U.S. Patent 4,289,838, the disclosure of which is hereby incorporated by reference, is particularly preferred. In broad terms, such compositions contain (a) solvent soluble, light sensitive diazonium components which can be any of the commonly used lithographic diazo compounds, or reaction or condensation products of such diazo compounds with agents therefor which do not materially impair the light sensitivity of the diazo, and (b) certain unsaturated monomers which are the products of about two equivalents of an organic di-isocyanate with about one equivalent of an active hydrogen containing organic compound such as an alcohol or amine, preferably a polyol, which products are subsequently reacted with an unsaturated compound which contains an active hydrogen, preferably in a hydroxyl group, available to react with the remaining isocyanate groups. The present invention, however, is not limited thereto.

In accordance with the present invention a light sensitive composition comprising a photocurable composition in combination with a photinitiator is characterised in that the said photoinitiator comprises dibenzoyl benzene.

The two benzoyl moieties can be positioned meta to one another or preferably are para to one another.

The dibenzoyl benzene is an effective photoinitiator and is substantially more resistant to sublimation when supported on a substrate than benzophenone, and so displays increased storage stability. Surprisingly the dibenzoyl benzene photo-initiator also causes the photocuring of the light sensitive composition to occur more rapidly. The dibenzoyl benzene may be substituted, such as, for instance, bis dialkylamino dibenzyl benzenes. The dibenzoyl benzene can be the only photoinitiator in the system, but in accordance with conventional practice, it is preferably used in combination with an internally acting photoinitiator such as Michler's ketone.

In a preferred embodiment, it has been found that particularly fast photocuring is achieved when the dibenzoyl benzene is used in combination with para-di[4-dimethylaminobenzoyl] benzene as the photoinitiator system. To our knowledge, the para-di[4-dimethylamino- benzoyl] benzene is a new compound and the method by which it is produced is described in the examples below.

The quantity of photoinitiator in the light sensitive composition is conventional. The amount of dibenzoyl benzene is generally the same as the amount that benzophenone has been employed in the past and the amount of through-cure photoinitiator such as Michler's ketone or the para-di[4-dimethylaminobenzoyl] benzene is generally the same as through-cure photoinitiators in conventional compositions.

The photocurable compositions can further contain fillers, binders, polymerization inhibitors, dyestuffs, color couplers, plasticizers, adhesion promotors, oxygen absorbing agents, and the like in varying quantities, in accordance with compositions well known in the art.

In order to further illustrate the invention, various examples are set forth below. In these examples, as throughout this specification and claims, all parts and percentages are by weight and all temperatures are in degrees centigrade unless otherwise specified.

## Example 1

This example describes the preparation of the new compound para-di[4-dimethylaminobenzoyl] benzene.

To a one liter flask were added 206 ml of N,N-dimethylaniline and 56 g of anhydrous aluminum chloride. The contents of the flask were heated slowly to reflux at which time 40 g of terephthaloyl chloride dissolved in 345 ml of N,N-dimethylaniline was added over a period of one to two hours while maintaining reflux. The contents of the flask were thereafter refluxed for a further three hours at which time the flask was cooled to room temperature and the reaction mixture poured into a mixture of 8 liters of water and 2 liters of hydrochloric acid at 10°C, with vigorous agitation. The desired product precipitated and was worked up by filtration, washing with 2-3 liters of water, air-drying and two recrystallizations from ethyl alcohol. The para-di[4-dimethylaminobenzoyl] benzene obtained was a greenish gray material and had a melting point of 210-212°C.

## Example 2

In order to compare and contrast the speed and through-cure achieved using various photoinitiators, a series of light sensitive coatings were prepared and hand-coated on a glass substrate. Each coating contained 94 parts of a light sensitive photocurable composition, one part of Michler's ketone and five parts of a second photoinitiator. Each coated glass plate was placed on a belt and conveyed past an ultraviolet lamp operated at 13.5 amps. The lamp was positioned about 3 to 4 inches above the belt web and generated about 200 watts per linear inch. The resulting exposed plates were then evaluated first by touch to determine whether they were tacky, which indicated the degree of curing. The plates were also evaluated by the standard methyl ethyl ketone resistance test to determine whether this solvent dissolved the coating, the test being an indication of the degree of cross-linking or insolubilization of the coating and can be equated to the length of run of the printing plate. The photoinitiator employed, belt speed, tackiness and methyl ethyl ketone (MEK) double rubs are set forth in the following table 2:

## Table 2

| Photoinitiator | Tackiness | MEK Double Rubs |
|---|---|---|
| (A) Belt Speed 200 ft/min | | |
| Benzophenone | slight | 3 |
| m-dibenzoyl benzene | slight | 3 |
| p-dibenzoyl benzene | none | 7-8 |
| Trans-1,2-dibenzoyl benzene | very | 2 |
| 1,3,5-tri-benzoyl benzene | slight | 6 |
| (B) Belt Speed 100 ft/min | | |
| Benzophenone | very slight | 3 |
| m-dibenzoyl benzene | none | 3-4 |

4

| | | |
|---|---|---|
| p-dibenzoyl benzene | none | 7-8 |
| Trans-1,2-dibenzoyl benzene | very slight | 1 |
| 1,3,5-tri-benzoyl benzene | none | 3 |
| (C) Belt Speed 75 ft/min | | |
| Benzophenone | none | 25 |
| m-dibenzoy benzene | none | 70-75 |
| p-dibenzoyl benzene | none | 150 |
| Trans-1,2-dibenzoyl benzene | none | 8 |
| 1,3,5-tri benzoyl benzene | none | 5 |

The foregoing table demonstrates that at a relatively slow belt speed of 75 ft/min., all of the photoinitiators cured the composition to the point where it was not tacky. As the belt speed was increased, however, the superiority of the dibenzoyl benzene photoinitiators became evident. At all speeds, the superiority of para-dibenzoyl benzene as measured by the MEK test was very evident.

Example 3

Two light sensitive compositions were prepared in accordance with the following formulas:

| | A | B |
|---|---|---|
| Unsaturated Acrylated Urethane | 93.6 | 93.6 |
| Michler's Ketone | 1.0 | 1.0 |
| Benzophenone | 5.0 | -- |
| p-Dibenzoylbenzene | -- | 5.0 |
| Victoria Pine Blue | 0.4 | 0.4 |

Coated glass plates were prepared and processed as in Example 2 except that the power of the UV lamp was 14.5 amps. The results are shown in the following Table.

| Belt Speed | A | B |
|---|---|---|
| 50 ft/min | 4 | 45 |
| 100 ft/min | 1 | 17 |

The foregoing data clearly demonstrates the superiority of the dibenzoyl benzene

Example 4

Following the procedure of Example 2, two light sensitive compositions were prepared which differed solely in the photoinitiator system. Each composition contained 93.6% urethane, five parts of p-dibenzoyl benzene, 0.4 part Victoria Pure Blue and one part of either Michler's ketone ("C") or p-di[4-dimethylamino-benzoyl] benzene ("D"). Coated glass plates were prepared as described in Example 2 and processed in the same manner. Table 3 shows the results achieved:

Table 3

| Belt speed = 100 ft/min | | |
|---|---|---|
| System | Tackiness | Double Rubs |
| C | none | 4 |
| D | none | 23 |

Various changes and modifications can be made in the products and process of the present invention without departing from the spirit and scope thereof. The various embodiments which have been set forth herein were for the purpose of further illustrating the invention but were not intended to limit it.

**Claims**

1. A light sensitive composition comprising a photocurable composition in combination · with a photoinitiator characterised in that the said photoinitiator comprises dibenzoyl benzene.

2. A light sensitive composition of claim 1 wherein said dibenzoyl benzene is p-dibenzoyl benzene.

3. A light sensitive composition of claim 1 or claim 2 wherein said photoinitiator further comprises p-di [4-dimethylamino benzoyl] benzene.

4. A light sensitive composition of any of the preceding claims supported on a substrate.

5. A cured composition obtained by curing a light sensitive composition wherein said light sensitive composition is that of any of the preceding claims.